(19)

Europäisches
Patentamt
European
Patent Office
Office européen
des brevets

(11)　**EP 3 354 745 B1**

(12)　　　　　　　　　　**EUROPEAN PATENT SPECIFICATION**

(45) Date of publication and mention
of the grant of the patent:
**05.08.2020　Bulletin 2020/32**

(21) Application number: **16848904.5**

(22) Date of filing: **21.09.2016**

(51) Int Cl.:
***C12Q 1/689*** *(2018.01)*

(86) International application number:
**PCT/KR2016/010511**

(87) International publication number:
**WO 2017/052178 (30.03.2017 Gazette 2017/13)**

(54) **METHOD FOR DIAGNOSING ATOPIC DERMATITIS**

VERFAHREN ZUR DIAGNOSE VON ATOPISCHER DERMATITIS

PROCÉDÉ DE DIAGNOSTIC DE LA DERMATITE ATOPIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2015　KR 20150134067
22.09.2015　KR 20150134129
20.09.2016　KR 20160120005**

(43) Date of publication of application:
**01.08.2018　Bulletin 2018/31**

(73) Proprietor: **Korea University Research and
Business Foundation
Seoul 02841 (KR)**

(72) Inventor: **KIM, Hee Nam
Seoul 07331 (KR)**

(74) Representative: **Cabinet Beau de Loménie
158, rue de l'Université
75340 Paris Cedex 07 (FR)**

(56) References cited:
**WO-A1-2014/138999　　KR-A- 20100 096 807**

- **DATABASE EMBL [Online] 2 June 2009
(2009-06-02), "Uncultured bacterium clone AIM_8
16S ribosomal RNA gene, partial sequence.",
XP002788418, retrieved from EBI accession no.
EM_STD:FJ719848 Database accession no.
FJ719848**

- **DATABASE EMBL [Online] 2 February 2010
(2010-02-02), "Uncultured bacterium clone
4-3O21 16S ribosomal RNA gene, partial
sequence.", XP002788419, retrieved from EBI
accession no. EM_STD:FJ680077 Database
accession no. FJ680077**
- **DATABASE Geneseq [Online] 7 May 2013
(2013-05-07), "Faecalibacterium prausnitzii 16-6-I
40 FAA 16S rRNA, SEQ 16.", XP002788420,
retrieved from EBI accession no. GSN:BAM28846
Database accession no. BAM28846 & WO
2013/037068 A1 (UNIV KINGSTON [CA];
KINGSTON GENERAL HOSPITAL [CA]; UNIV
GUELPH [CA];) 21 March 2013 (2013-03-21)**
- **DATABASE GNPD [Online] MINTEL; 15 April 2015
(2015-04-15), anonymous: "Uncultured
Faecalibacterium sp. clone OTU4591 16S
ribosomal RNA gene, - Nucleotide - NCBI",
XP55519777, retrieved from www.gnpd.com
Database accession no. KM976866**
- **DATABASE GNPD [Online] MINTEL; 28
September 2013 (2013-09-28), anonymous:
"Uncultured Faecalibacterium sp. clone 8167 16S
ribosomal RNA gene", XP055374003, retrieved
from www.gnpd.com Database accession no.
KF508452.1**
- **MARCO CANDELA ET AL: "Unbalance of
intestinal microbiota in atopic children", BMC
MICROBIOLOGY, BIOMED CENTRAL LTD, GB,
vol. 12, no. 1, 6 June 2012 (2012-06-06), pages 1-9,
XP021126153, ISSN: 1471-2180, DOI:
10.1186/1471-2180-12-95**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

EP 3 354 745 B1

- MIREIA LOPEZ-SILES ET AL: "Mucosa-associated Faecalibacterium prausnitzii and Escherichia coli co-abundance can distinguish Irritable Bowel Syndrome and Inflammatory Bowel Disease phenotypes", INTERNATIONAL JOURNAL OF MEDICAL MICROBIOLOGY, vol. 304, no. 3-4, 1 May 2014 (2014-05-01) , pages 464-475, XP055319224, DE ISSN: 1438-4221, DOI: 10.1016/j.ijmm.2014.02.009
- SONG HAN ET AL: "Faecalibacterium prausnitzii subspecies-level dysbiosis in the human gut microbiome underlying atopic dermatitis", JOURNAL OF ALLERGY AND CLINICAL IMMUNOLOGY, ELSEVIER, AMSTERDAM, NL, vol. 137, no. 3, 1 October 2015 (2015-10-01), pages 852-860, XP029451330, ISSN: 0091-6749, DOI: 10.1016/J.JACI.2015.08.021

- DATABASE NUCLEOTIDE [Online] 28 September 2013 NCBI, XP055374003 Database accession no. KF508452.1
- DATABASE NUCLEOTIDE [Online] 15 April 2015 XP055519777 Retrieved from NCBI Database accession no. KM976866
- CANDELA ET AL.: 'Unbalance of Intestinal Microbiota in Atopic Children' BMC MICROBIOLOGY vol. 12, no. 95, 2012, pages 1 - 9, XP021126153
- SONG ET AL.: 'Faecalibacterium Prausnitszii Subspecies-level Dysbiosis in the Human Gut Microbiome Underlying Atopic Dermatitis' THE JOURNAL OF ALLERGY AND CLINICAL IMMUNOLOGY vol. 137, no. 3, March 2016, pages 852 - 860, XP029451330

**Description**

TECHNICAL FIELD

The following description relates to a biomarker composition that includes a nucleotide sequence of SEQ ID NO: 1 or SEQ ID NO: 2 as an active ingredient, a diagnostic kit that includes the biomarker composition. The present invention relates to an information providing method that includes an operation of recognizing a nucleotide sequence including SEQ ID NO: 1 or SEQ ID NO: 2, wherein the recognizing of the nucleotide sequence is performed to diagnose atopic dermatitis (AD).

BACKGROUND ART

Atopic dermatitis (AD) is a chronic inflammatory skin disorder, and its incidence is increasing globally. AD is a chronic recurrent inflammatory skin disorder that mostly starts in infancy or childhood, and is accompanied by pruritus, xeroderma, or severe inflammation.

Although symptoms of AD vary with age, lesions are often accompanied by pruritus. In early childhood, the symptoms may appear on cheeks, foreheads, heads, trunks, arms or legs. In late childhood, symptoms mainly appear on intertriginous areas of arms, legs or earlobes.

In the United States, up to 25% of children and 2% to 3% of adults suffer from AD. However, causes of AD are not yet known. Although it is not possible to accurately explain the causes, environmental factors or genetic factors are regarded as major causes in view of the fact that cases are increasing mainly in countries industrialized in recent years.

There is no specific test for diagnosis of AD, however, tests to identify factors that aggravate AD are available. Tests to identify allergens that cause AD may include skin prick tests, serum-specific immunoglobulin E tests, oral food challenge tests, and bacterial culture tests.

Although the immediate cause and pathogenesis of AD are still unclear, skin damage in patients is regarded to be primarily caused through a mechanism involving the overproduction of proinflammatory cytokines that cause inflammation when a TH 2 type immune response reacts in a hypersensitive manner to a general antigen. Moreover, other characteristics, such as simplification of the microbial community on the skin and an increase in *Staphylococcus aureus,* have been observed. It has also been reported that specific bacteria in the intestines may be associated with an atopic disease.

Despite the above research results, the microbiological cause of the dysregulation of inflammation that is the basis of atopy remains unknown. Thus, an important issue in this field is to be able to determine the biological cause of atopy and to diagnose it to clarify that it has indeed developed.

DISCLOSURE OF INVENTION

TECHNICAL SUBJECTS

The disclosure provides a biomarker composition including a nucleotide sequence of SEQ ID NO: 1 or SEQ ID NO: 2 as an active ingredient.

The disclosure also provides a biomarker composition for detecting a subspecies of *Faecalibacterium prausnitzii* (F06).

The disclosure also provides a biomarker composition for diagnosis of atopic dermatitis (AD).

The disclosure also provides a biomarker composition including a nucleotide sequence having a sequence identity of 97% or higher to SEQ ID NO: 1 or SEQ ID NO: 2 as an active ingredient.

The disclosure also provides a diagnostic kit including a biomarker composition including a nucleotide sequence of SEQ ID NO: 1 or SEQ ID NO: 2 as an active ingredient.

The disclosure also provides a diagnostic kit including a biomarker composition for detecting a subspecies of *F. prausnitzii* (F06), and more particularly, a diagnostic kit including a biomarker composition for diagnosis of AD.

The disclosure also provides a diagnostic kit including a biomarker composition including a nucleotide sequence having a sequence identity of 97% or higher to SEQ ID NO: 1 or SEQ ID NO: 2 as an active ingredient.

The invention, example embodiments provides a method of providing information, the method including providing an isolated nucleic acid sample; and recognizing a nucleotide sequence including SEQ ID NO: 1 or SEQ ID NO: 2, wherein the recognizing of the nucleotide sequence is performed to diagnose AD.

The recognizing of the nucleotide sequence including SEQ ID NO: 1 or SEQ ID NO: 2 may include detecting a subspecies of *Faecalibacterium prausnitzii.*

According to the disclosure, the nucleotide sequence including SEQ ID NO: 1 or SEQ ID NO: 2 may be a nucleotide sequence having a sequence identity of 97% or higher to SEQ ID NO: 1 or SEQ ID NO: 2.

TECHNICAL SOLUTIONS

Hereinafter, example embodiments will be described in detail with reference to the accompanying drawings, and like reference numerals in the drawings refer to like elements throughout.

Various modifications may be made to the following example embodiments. Here, the example embodiments are not construed as limited to the disclosure and should be understood to include all changes, equivalents, and replacements within the idea and the technical scope of the disclosure.

The terminology used herein is for the purpose of describing particular example embodiments only and is not to be limiting of the examples. As used herein, the singular forms are intended to include the plural forms as well, unless the context clearly indicates otherwise. It should be further understood that the terms "include/comprise" and/or "have" when used in this specification, specify the presence of stated features, integers, steps, operations, elements, components and/or combinations thereof, but do not preclude the presence or addition of one or more other features, integers, steps, operations, elements, components, and/or groups thereof.

Unless otherwise defined, all terms including technical and scientific terms used herein have the same meaning as commonly understood by one of ordinary skill in the art to which example embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

Also, in the following description with reference to the accompanying drawings, like reference numerals refer to like constituent elements, and a repeated description related thereto will be omitted. When it is determined detailed description related to a related known function or configuration they may make the purpose of the example embodiments unnecessarily ambiguous in describing the example embodiments, the detailed description will be omitted here.

Hereinafter, the terms used in the present disclosure are described.

The term "biomarker," as used herein, generally refers to an indicator to identify a change in a body using proteins, DNA, RNA, metabolites, and the like.

The term "genome," as used herein, refers to a cluster of genes that include all genetic information for a human body, and the term "microbiome," which is called the "second genome," refers to all genetic information of microorganisms coexisting in the body. In particular, the term "gut microbiome" refers to all genetic information of microorganisms existing in the body, in particular, the intestines. Recently, the interest in the gut microbiome has increased because it has been reported that various microorganisms in the human body influence metabolism regulation, digestive ability, or various diseases as well as influence all functions of the human body such as genetic modifications due to environmental changes.

The term "diagnosis," as used herein, may include a prediction or diagnosis of the outcome or state of an individual, the state or outcome, the progression of a condition, or a prediction of the response to a specific treatment. In example embodiments of the present disclosure, typical technologies of immunology, biochemistry, molecular biology, microbiology, cell biology, genomics, and recombinant DNA within the skill of the related art may be employed, unless otherwise indicated. The terms "individual," "patient," or "object," as used herein, may include humans and other mammals.

Throughout the present specification and drawings, the term "AD" indicates an atopic dermatitis, the term "SCFA" indicates short-chain fatty acid, the term "OTU" indicates operational taxonomic unit, the term "GalNAc" indicates N-acetylgalactosamine, and the term "KO" indicates Kyoto Encyclopedia of Genes and Genomes (KEGG) Orthology.

According to an aspect of the disclosure, a biomarker composition including a nucleotide sequence of SEQ ID NO: 1 or SEQ ID NO: 2 as an active ingredient may be provided.

According to the disclosure, the biomarker composition including the nucleotide sequence of SEQ ID NO: 1 or SEQ ID NO: 2 as an active ingredient may be used to determine the presence or absence of various diseases including the nucleotide sequence of SEQ ID NO: 1 or SEQ ID NO: 2 from samples of various individuals with a disease or suspected of having a disease.

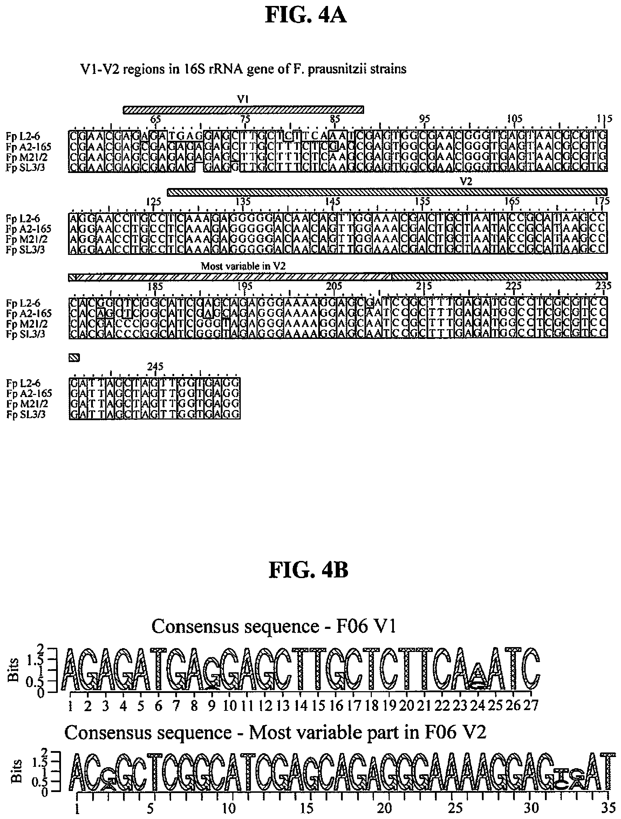

The nucleotide sequences of SEQ ID NO: 1 and SEQ ID NO: 2 are provided below (see FIGS. 4A through 4D).

    SEQ ID NO: 1-AGAGATGAGGAGCTTGCTCTTCAAATC
    SEQ ID NO: 2-ACGGCTCGGCATCGAGCAGAGGGAAAAGGAGTGAT

Referring to FIG. 4B, a nucleotide sequence of an F06 VI region is not limited to SEQ ID NO: 1 and may have G or A at position 9, C or T at position 13, and A or G at position 24 ((position 9(G, A), position 13(C, T), position 24(A, G)). In FIG. 4B, a nucleotide sequence of an F06 V2 region is not limited to SEQ ID NO: 2 and may have G or A at position 3, C or G at position 5, G or C at position 16, A or G at position 20, T or C at position 32, and G or A at position 33 (position 3(G, A), position 5(C, G), position 16(G, C), position 20(A, G), position 32(T, C), position 33(G, A)).

For example, the biomarker composition of the disclosure may be a biomarker composition for detecting a subspecies of *F. prausnitzii* (F06).

According to the disclosure, the nucleotide sequence of SEQ ID NO: 1 or SEQ ID NO: 2 may be used as a biomarker composition for detecting a subspecies of *F. prausnitzii* (F06) in a microbiome. A biomarker composition including the nucleotide sequence may be used to diagnose a variety of diseases related to the subspecies of *F. prausnitzii* (F06).

As another example, the biomarker composition of the description may be a biomarker composition for diagnosing AD.

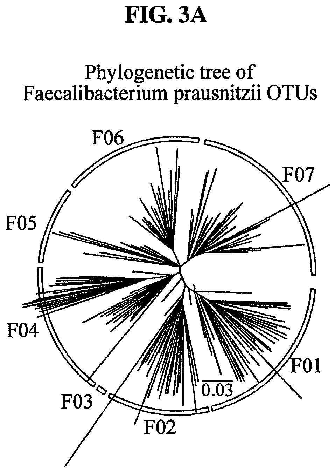

Based on the V1-V2 sequence indicating the nucleotide sequence of SEQ ID NO: 1 or SEQ ID NO: 2, a great increase in the AD microbiome was confirmed (see FIGS. 3A through 3C). In particular, an increase in F06 in the AD microbiome was observed to be common for all ages but was especially prominent in the age group that was less than one year old, which indicates that the subspecies of *F. prausnitzii* (F06) is involved in an onset of AD. The biomarker composition was determined to be suitable for the diagnosis of AD, as described above, and may also be used to diagnose all diseases involving the nucleotide sequence of SEQ ID NO: 1 or SEQ ID NO: 2.

According to another aspect of the disclosure, the biomarker composition may be a biomarker composition including a nucleotide sequence having a sequence identity of 97% or higher to SEQ ID NO: 1 or SEQ ID NO: 2 as an active ingredient.

Actually, among Koreans, differences have been found in the nucleotides at positions 9, 13, and 24 of SEQ ID NO: 1 and positions 3, 5, 16, 20, 32, and 33 of SEQ ID NO: 2. Thus, each of the nucleotides at positions 9, 13, and 24 of SEQ ID NO: 1 and positions 3, 5, 16, 20, 32, and 33 of SEQ ID NO: 2 may be variously replaced. Desirably, SEQ ID NO: 1 or SEQ ID NO: 2 may be replaced with one having a sequence identity of 97% or higher to the entire sequence. For example, G at position 9 of SEQ ID NO: 1 may be replaced with A, C at position 13 may be replaced with T, and A at position 24 may be replaced with G. In addition, G at position 3 of SEQ ID NO: 2 may be replaced with A, C at position 5 may be replaced with G, G at position 16 may be replaced with C, A at position 20 may be replaced with G, T at position 32 may be replaced with C, and G at the position 33 may be replaced with A. In other words, the biomarker composition may include, for example, a biomarker composition including a nucleotide sequence having a sequence identity that is 97% or higher to SEQ ID NO: 1 or SEQ ID NO: 2 as an active ingredient so that it is within the range of the homogeneous diversity of 16S rRNA genes of typical bacteria.

According to another aspect of the disclosure, a diagnostic kit including a biomarker composition that includes a nucleotide sequence of SEQ ID NO: 1 or SEQ ID NO: 2 as an active ingredient may be provided.

According to the disclosure, a diagnostic kit for diagnosis of a composition including a nucleotide sequence of SEQ ID NO: 1 or SEQ ID NO: 2, and desirably, a diagnostic kit for diagnosis of AD may be provided when a nucleic acid sample isolated from an individual is provided. The diagnostic kit may be used for any type of disease related to the subspecies of *F. prausnitzii* (F06). Moreover, the diagnostic kit, after being determined to be suitable for the diagnosis of AD, may also be used to diagnose all diseases involving the nucleotide sequence of SEQ ID NO: 1 or SEQ ID NO: 2.

In addition, a diagnostic kit including a biomarker composition including a nucleotide sequence having a sequence identity of 97% or higher to SEQ ID NO: 1 or SEQ ID NO: 2 as an active ingredient may be provided.

According to the invention, a method of providing information is provided. The method includes providing an isolated nucleic acid sample, and recognizing a nucleotide sequence that includes SEQ ID NO: 1 or SEQ ID NO: 2 wherein the recognizing of the nucleotide sequence is performed to diagnose AD.

When a nucleic acid sample isolated from an individual is provided, a nucleotide sequence including SEQ ID NO: 1 or SEQ ID NO: 2 is recognized, and useful information required for an AD diagnosis is provided.

The recognizing of the nucleotide sequence including SEQ ID NO: 1 or SEQ ID NO: 2 may include detecting a subspecies of *Faecalibacterium prausnitzii.*

A method of providing information for detection of a subspecies of *Faecalibacterium. prausnitzii* by recognizing a nucleotide sequence including SEQ ID NO: 1 or SEQ ID NO: 2 when a nucleic acid sample isolated from an individual is provided, may be provided. A method of providing information for diagnosis of AD associated with the above bacteria is provided.

According to the disclosure, the nucleotide sequence including SEQ ID NO: 1 or SEQ ID NO: 2 may be a nucleotide sequence having a sequence identity of 97% or higher to SEQ ID NO: 1 or SEQ ID NO: 2.

Actually, among Koreans, differences have been found in nucleotides at positions 9, 13, and 24 of SEQ ID NO: 1 and positions 3, 5, 16, 20, 32, and 33 of SEQ ID NO: 2. Thus, each of the nucleotides at positions 9, 13, and 24 of SEQ ID NO: 1 and positions 3, 5, 16, 20, 32, and 33 of SEQ ID NO: 2 may be variously replaced. Desirably, SEQ ID NO: 1 or SEQ ID NO: 2 may be replaced with one having a sequence identity of 97% or higher to the entire sequence. For example, G at position 9 of SEQ ID NO: 1 may be replaced with A, C at position 13 may be replaced with T, and A at position 24 may be replaced with G. In addition, G at position 3 of SEQ ID NO: 2 may be replaced with A, C at position 5 may be replaced with G, G at position 16 may be replaced with C, A at position 20 may be replaced with G, T at position 32 may be replaced with C, and G at position 33 may be replaced with A. In other words, the biomarker composition may include, for example, a biomarker composition including a nucleotide sequence having a sequence identity that is 97% or higher to SEQ ID NO: 1 or SEQ ID NO: 2 as an active ingredient so that it is within the range of the homogeneous diversity of 16S rRNA genes of typical bacteria.

EFFECT OF THE INVENTION

According to the disclosure, a biomarker composition that includes a nucleotide sequence of SEQ ID NO: 1 or SEQ ID NO: 2 as an active ingredient, and a diagnostic kit that includes the biomarker composition may be effectively used for diagnosis and treatment of atopic dermatitis (AD) with a high incidence worldwide.

According to example embodiments, whether a subspecies of *F. prausnitzii* (F06) having a specific nucleotide sequence is present may be usefully determined, and in particular, whether AD is likely to occur may be easily and accurately diagnosed before AD occurs, and thus the present disclosure may be very useful for individuals both before and after AD occurs.

BRIEF DESCRIPTION OF DRAWINGS

FIG. 1A illustrates an $\alpha$ diversity profile of a gut microbiome, FIG. 1B illustrates a $\beta$ diversity profile of the gut microbiome, FIG. 1C illustrates an analysis of enterotypes, and FIG. ID illustrates a relative abundance (%) of *Ruminococcus, Parabacteroides, Faecalibacterium* and *EscherichialShigella.*

FIG. 2A is a histogram showing results from an RFE-SVM analysis, and FIG. 2B illustrates a list of bacterial clades including top discriminatory OTUs.

FIG. 3A illustrates a phylogenetic tree of *F. prausnitzii* OTUs to select operational taxonomic units (OTUs) for distinguishing between atopic patients and non-atopic controls, FIG. 3B illustrates an OUT abundance, and FIG. 3C illustrates an F06 abundance.

FIG. 4A illustrates a VI-V2 region of a 16S rRNA gene of each *F. prausnitzii* strain, FIG. 4B illustrates a consensus sequence of an F06 VI region, FIG. 4C illustrates all F06 454 reads, and FIG. 4D illustrates a relative abundance of a VI sequence.

FIG. 5A illustrates a heat map of significantly altered functions in an atopic dermatitis (AD) microbiome, and FIG. 5B illustrates sequences of genes of five strains of *F. prausnitzii,* that is, A2-165, M21/2, L2-6, S3L/3 and KLE1255.

FIG. 6A is a graph illustrating analyses of SCFAs of an experimental group and a control group, and FIG. 6B illustrates a comparison of butyryl CoA: acetate CoA-transferase gene promoter activity from *F. prausnitzii* strains.

FIG. 7 illustrates a proposed model of AD associated with a dysfunction of *F. prausnitzii.*

FIG. 8A is a Venn diagram illustrating common genes or unique genes in strains, and FIG. 8B illustrates Illumina reads of each gene group.

MODE FOR CARRYING OUT THE INVENTION

Hereinafter, the present disclosure will be described in detail based on example embodiments.

The inventor of the present disclosure has estimated that specific bacteria in the intestines may be associated with an atopic disease and has studied the correlation between intestinal bacteria and atopy for a long period of time. To demonstrate the correlation, "90" atopic patients (experimental group) and "42" subjects without atopic symptoms (control group) participated in the present study, and the 16S rRNA genes and metagenomes of the gut microbial community of "132" subjects in total were analyzed. Reference gene data from the Human Microbiome Project and the KEGG Orthology database were used for the metagenomic analysis. The severity of the symptoms of atopic patients was evaluated in advance using a SCORAD system, and the control group was formed of individuals who never had atopic dermatitis (AD). Both the experimental group and the control group were not exposed to antibiotics within the six months prior to providing fecal samples. The experimental group was composed of outpatients at the Anam Hospital of Korea University, and the present study was approved by the Ethics Committee. Fecal samples of approximately 5 g were obtained from the experimental group and the control group and stored at -80°C until DNA was isolated. Short-chain fatty acids (SCFAs) in the fecal samples were compared and analyzed using a gas chromatographic mass spectrometer.

Example 1: DNA isolation

Samples were ground in liquid nitrogen with a mortar and pestle, and 400 mg of the ground samples was transferred to four microcentrifuge tubes. Each of the tubes contained 500 $\mu$l of a solution including 0.1 mm-diameter zirconia/silica beads (BioSpec products, Bartlesville, OK, USA), 500 $\mu$l of an extraction buffer (200 mM Tris [pH 8.0], 200 mM NaCl, 20 mM EDTA), 210 $\mu$l of 20% SDS, and a phenol:chloroform:isoamyl alcohol mixture (25:24:1, pH 7.9).

The samples were ground with a bead beater for 2.5 minutes at room temperature, and the bacterial contents were released by disrupting bacterial cell envelopes. DNA was isolated from the supernatant using the phenol/chloroform/isoamyl alcohol (25:24:1, pH 7.9) and precipitated with isopropanol. DNA pellets were dried and dissolved in a TE buffer (10 mM Tris [pH 8.0], 1 mM EDTA), and the concentration was adjusted to 100 ng/$\mu$l.

Example 2: Sequence analysis of V1-V2 region of 16S rRNA gene

A PCR reaction targeting the VI-V2 region of the 16S rRNA gene was conducted with each DNA sample in a 25-$\mu$l reaction mixture including 100 ng of template DNA, 5 $\mu$l of a HotStar PCR buffer (Qiagen, Germantown, MD, USA), 1 U of HotStarTaq DNA polymerase (Qiagen), and 0.4 $\mu$mol/l each of a forward primer and a reverse primer. The forward primer, 5'-<u>CGTATCGCCTCCCTCGCGCCATCAG</u>NNNNNNNN**TC***AGGAGTTTGATCCTGGCTC AG-3'*, was formed of a 454 titanium adapter A (underlined nucleotides), a unique 8-base barcode (shown with eight Ns), a linker (a dinucleotide **TC**), and universal bacterial primer 8F. The reverse primer, 5'-*<u>CTATGCGCCTTGCCAGCCCGCTCAG</u>*NNNNNNNN**CAT-GCTGCCTCCCGTAGGAG** *T*-3', was formed of a 454 titanium adapter B (underlined), a unique 8-base barcode (Ns), a linker **(CA),** and a broad-range bacterial primer 338R (in italics).

A DNA amplification was conducted at 95°C for 15 minutes, followed by 34 cycles of 95°C for 30 seconds, 52°C for 30 seconds, and 72°C for 1 minute, with a final extension at 72°C for 5 minutes. PCR products were obtained using a QIAquick PCR Purification Kit (Qiagen). Amplicons were quantified with the PicoGreen Assay and diluted to $1 \times 10^9$ molecules/$\mu$l in the TE buffer. A sequencing library was constructed for 454FLX titanium pyrosequencing using an equal amount of each product.

Example 3: Analysis of 454 sequencing reads

Barcode-tagged VI-V2 region sequence reads of the 16S rRNA gene were analyzed using a QIIME pipeline. Sequences lacking either the 5' or 3' end sequences, or sequences including at least one ambiguous nucleotide were excluded.

All primer sequences were removed and trimmed sequences shorter than 250 bp were excluded. The other sequences were classified into operational taxonomic units (OTUs) using a CD-HIT based on a 97% sequence similarity and were taxonomically identified using an RDP classifier based on a confidence score of 0.5%. Chimera sequences were removed using a ChimeraSlayer and a BlastFragment in the QIIME pipeline. The Shannon index was calculated to know the $\alpha$-diversity of the gut microbiota, and an unweighted UniFrac or Bray-Curtis distance metrics for a $\beta$-diversity analysis were constructed using QIIME scripts.

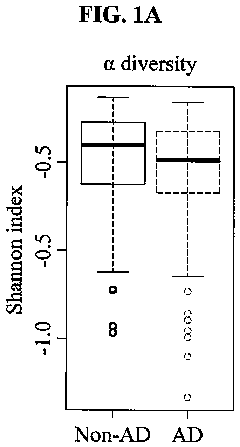

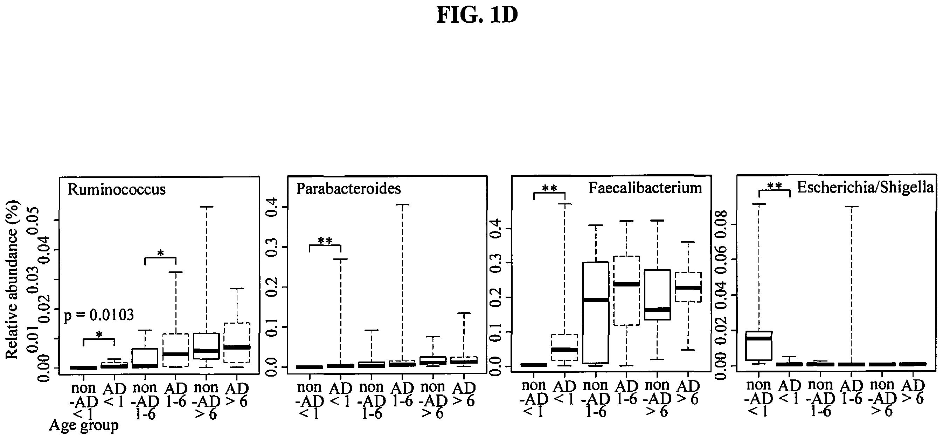

An enterotype analysis used a genus-level distance matrix constructed based on the Jensen-Shannon divergence algorithm, and was conducted using the partitioning around medoids (PAM) clustering algorithm. The Calinski-Harabasz index was calculated to know the optimal number of an enterotype cluster (see FIGS. 1A through ID).

454 sequencing reads were analyzed through an OTU analysis using the CD-HIT or analyzed by constructing taxon-independent OTUs using a separate ESPRIT. An OTU abundance matrix formed by the above OTUs was used to identify the most discriminatory OTU between atopic patients and non-atopic controls using a recursive feature elimination support vector machine (RFE-SVM). OTUs that were absent in at least 30% of samples were excluded from data to reduce the noise level. A leave-one-out cross-validation was used to measure the prediction accuracy for the identification of the AD-microbiota using a selected set of discriminatory OTUs.

Example 4: Whole-genome sequencing and sequence analysis

Whole-genome sequences of eight fecal samples (four samples each from the experimental group and the control group) were obtained using the Illumina HiSeq2000 platform. A DNA library with a fragment length of approximately 400 bp was prepared, and paired-end reads of 101 bp were obtained.

Sequence analysis was conducted with a CLC Genomics Workbench. Low-quality reads were excluded based on the following: all sequences with a quality score less than 0.05 or a sequence with at least two ambiguous nucleotide sequences. Sequences shorter than 15 bp were also excluded. Functions of the metagenome were analyzed using HUMAnN v0.99. All *Bacteroides* and *Faecalibacterium* genomes (bfg, bhl, bsa, bxy, bdo, bdh, bacc, fpr, and fpl) were added to a KEGG Orthology (KO) database v54 and used for analysis. A translated BLAST search of the Illumina reads against the KO database was conducted using USEARCH v8.0. Results were processed with HUMAnN, and normalized orthologous gene family abundances were compared between the experimental group and control group.

Example 5: Pangenomic analysis of five *F. prausnitzii* strains

Genome sequences of five *F. prausnitzii* strains were downloaded from the Human Microbiome Project (HMP) website (http://www.hmpdacc.org/) to perform a pangenomic analysis. Coding DNA sequences (CDSs) from the strains were compared using BLASTP and clustered into discrete orthologs based on amino acid level similarity and matching regions of 70% or greater. The CDSs were organized using the KO database based on BLASTP hits, with an e-value cutoff of 1e-5.

Whole-metagenome reads from Illumina HiSeq2000 sequencing were mapped on the genomes of the five *F. prausnitzii* strains using CLC Genomics Workbench. As a result, 10.14% of the Illumina reads were mapped on the *F. prausnitzii* genomes.

Example 6: Measurement of SCFAs in samples

Samples were ground in liquid nitrogen with a mortar and pestle, and approximately 0.1 g of the ground samples was transferred to a 2-mL microfuge tube. An internal standard (60 μL of 0.25 mmol/l 4-methylvaleric acid) was added to a tube including a ground sample and used to measure SCFAs. After acidification of the samples with 20 μL of 33% hydrogen chloride (HCl), diethyl ether was added, followed by vortexing for 10 minutes. The diethyl ether layer was centrifuged and transferred to a new tube. The same procedure was repeated one more time, and then, the diethyl ether phases from two extractions were mixed. 60 μl of the extract and 20 μL of *N-tert*-butyldimethylsilyl-*N*-methyltrifluoroacetamide were mixed in a glass insert in a gas chromatography autosampler vial and incubated for 2 hours at room temperature. Gas chromatography mass spectrometry was conducted using the prepared samples. Ratios of D-lactate and L-lactate were acquired using a Megazyme enzymatic kit (Megazyme, Ireland).

Frozen samples (0.1 g) were dissolved in 1 ml of 0.1 M triethanolamine buffer (pH 9.15) and centrifuged at 14,000 for 10 minutes at 4°C. The supernatant was precipitated with 6.1 N trichloroacetic acid (10% final concentration) and centrifuged at 4500g for 20 minutes at 4°C. The precipitated supernatant was used to measure a the lactate level.

Example 7: Promoter activity analysis

To compare the promoter activity of the butyryl CoA:acetate CoA-transferase gene in five *F. prausnitzii* strains (FP2_20620 for strain L2-6, FAEPRAA2165_01575 for strain A2-165, HMPREF9436_00973 for strain KLE1255, FPR 29560 for strain SL3/3, and FAEPRAM212_02812 for strain M21/2), a *lacZY* fusion was made to the promoter of each gene using the promoter probe vector pLKC481, and plasmid constructs were transferred to *Escherichia coli* DH5α. *E. coli* strains including different pLKC481 constructs were grown in an LB broth to the mid-log phase ($OD_{600}$ = 0.7) and left on ice for 20 minutes. 2 ml of each sample was harvested, and pellets were resuspended in a Z buffer. A β-galactosidase activity representing the promoter activity was measured using O-nitrophenyl-β-D-galactoside as a substrate and expressed as Miller units calculated using the following expression:

$$1000 \times (OD_{420} - 1.75 \times OD_{550}) / (\text{Time of incubation [min]} \times \text{Volume [ml]} \times OD_{600})$$

Hereinafter, the present disclosure will be further described based on the drawings.

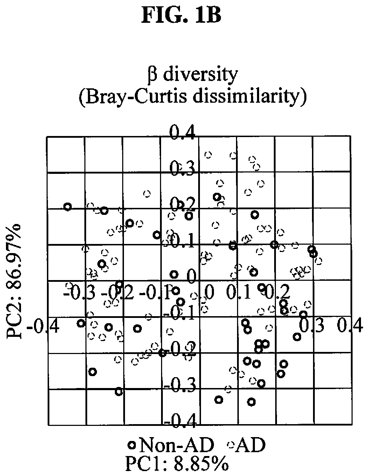

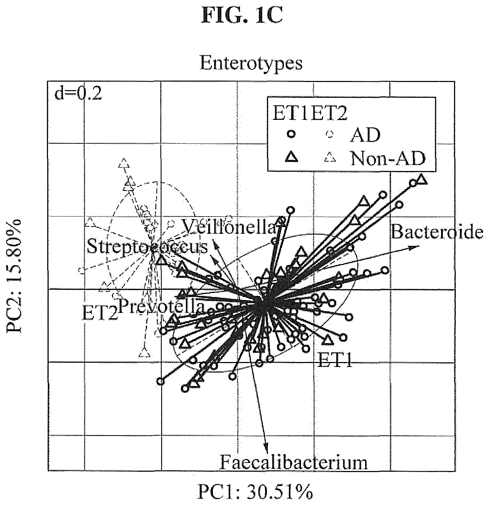

FIG. 1A illustrates an α diversity profile of a gut microbiome, FIG. 1B illustrates a β diversity profile of the gut microbiome, FIG. 1C illustrates an analysis of enterotypes, and FIG. ID illustrates a relative abundance (%) of *Ruminococcus, Parabacteroides, Faecalibacterium* and *EscherichialShigella.*

DNA representing the gut microbiome from "90" subjects in the experimental group and "43" subjects in the control group was isolated from fecal samples, and V1-V2 region in 16S rRNA genes were amplified and sequenced using 454 technology. The sequence reads were found to have an average sequencing depth of 6,604 reads after low-quality sequences were removed. No notable difference was found in a microbial α diversity between the microbiomes of atopic patients and those of non-atopic controls (see a graph of FIG. 1A). Moreover, no notable difference was found in microbial β diversity (composition) between the atopic patients and non-atopic controls (see a graph of FIG. 1B). The microbiome may be designated as a specific enterotype based on major bacteria groups. Although the "132" microbiomes were classified as one of two enterotypes, that is, *Bacteroides* or *Prevotella,* AD was not classified as one of the enterotypes (see a graph of FIG. 1C). Thus, there was no marked overall change in microbiomes in AD. However, a meaningful difference between the experimental group and the control group was observed for a specific bacterial clade in groups divided by age (see the graph of FIG. 1B). Although there have been reports of an increase in bacteria belonging to the family Enterobacteriaceae and decreases in bacteria belonging to *Lactobacillus* and *Bifidobacterium* species in patients with AD, the above trend was not observed in the present experiment. In addition, bacteria reported to be associated with atopy in previous studies may include bacteria with increased opportunistic growth in an unhealthy gut environment and are not directly associated with atopy. Bacteria associated with atopy may include bacteria that directly have pathogenicity or bacteria that simply have a growth advantage in a damaged gut environment. Pathogenic bacteria that play an important role in atopy may be a single species or a consortium of specific bacteria, and may be increased in all atopic environments. Bacteria that simply utilize a disease environment may include various types of bacteria. As a whole, the above bacteria may seem to be increased in atopic environments, however, may be absent in individual samples. Considering the above pattern, most bacteria increased in atopy microbiomes (see the graphs of FIGS. 1A through ID) are highly likely to be bacteria that simply utilize atopic environments. However, because a few causative bacteria responsible for atopy are present but are mixed with these other bacteria, it may be difficult to distinguish the causative bacteria from the other bacteria due to the overwhelming presence of the other bacteria.

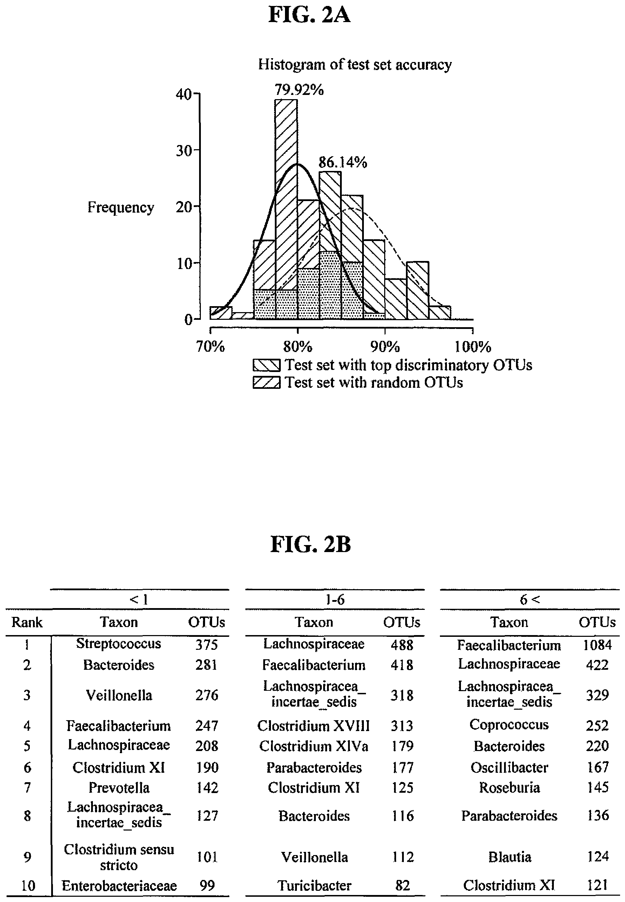

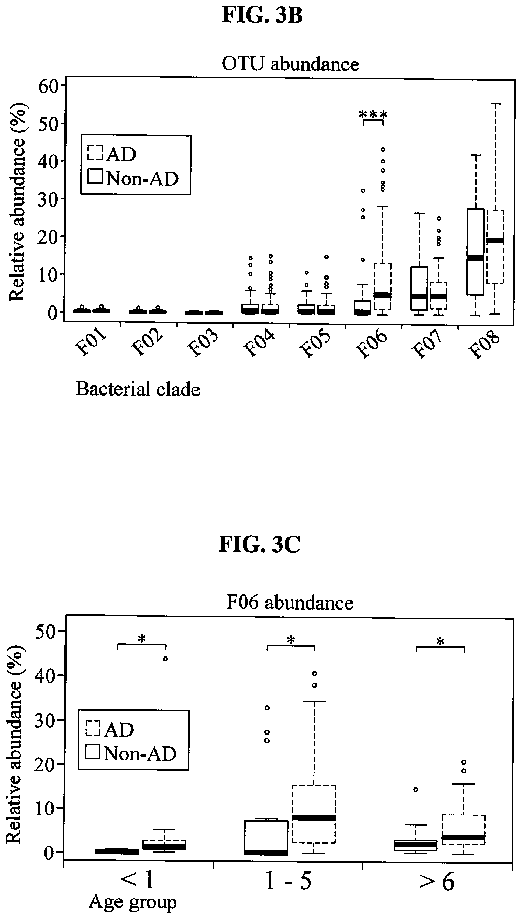

FIG. 2A is a histogram showing results from an RFE-SVM analysis, and FIG. 2B illustrates a list of bacterial clades including top discriminatory OTUs. FIG. 3A illustrates a phylogenetic tree of *F. prausnitzii* OTUs to select operational taxonomic units (OTUs) for distinguishing between atopic patients and non-atopic controls, FIG. 3B illustrates an OUT abundance, and FIG. 3C illustrates an F06 abundance.

The RFE-SVM analysis was conducted to select the top discriminatory OTUs to distinguish between atopic patients and non-atopic controls. For the above analysis, OTUs were constructed using ESPRIT, which is based on a taxonomy-independent hierarchical classification (at a 0.03 distance level that generally matches the species level). One-half of all data were randomly selected and used as a training set for the RFE-SVM analysis to select discriminatory OTUs. The other half of the data were used to test the atopy identification abilities of the OTUs. The OTUs showed an accuracy of 88.14% in the atopy identification abilities, in comparison to randomly selected OTUs with an accuracy of 79.92% (see FIG. 2A). It was found that a large number of OTUs among the top discriminatory OTUs belonged to a few clades and were common among all ages (see FIG. 2B). Among these, in particular, the *Faecalibacterium* species was shown to significantly increase in the atopy microbiomes (see the graph of FIG. ID). Currently, all *Faecalibacterium* species are grouped into a single species, *F. prausnitzii*, although two physiologically distinct phylotypes are present.

Atopy-related subspecies of *F. prausnitzii*

*F. prausnitzii* was markedly present in the atopy microbiome. Because *F. prausnitzii* is one of most significant species in an RFE-SVM model and is one of most abundant species in the human gut microbiome, an experiment was conducted to determine if it performs an important function in both normal human physiology and AD. Also, because *F. prausnitzii* is a single species, it was relatively easy to study the composition of the clade. The study was conducted based on the possibility of a specific subspecies being more associated with AD than all species. ESPRIT OTUs annotated as *F. prausnitzii* were clustered into seven clades in the phylogenetic tree. Because the phylogenetic tree is based on the V1-V2 sequences, it is possible that each clade may be further divided when the entire sequence of the 16S rRNA gene is applied. F06 was one of the clades that was significantly increased in the AD microbiome (see FIGS. 3A through 3C). An increase in F06 in the AD microbiome was common among all ages and was particularly prominent in the less than one-year-old group (see FIGS. 3A through 3C). Because AD mainly starts before one year of age, this result shows that an *F. prausnitzii* subspecies is involved in the onset of the above disease.

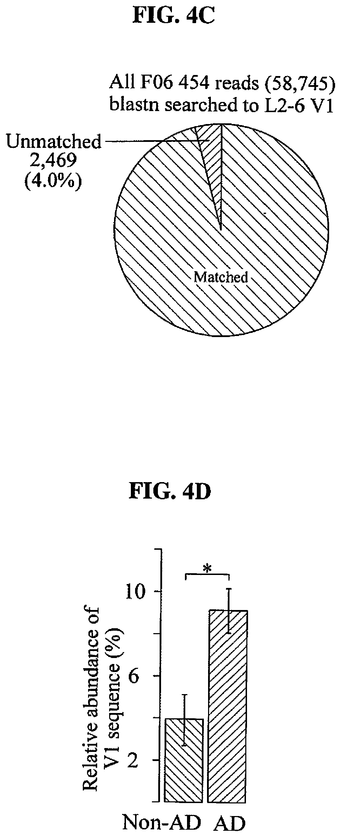

FIG. 4A illustrates the V1-V2 region of the 16S rRNA gene of each *F. prausnitzii* strain, FIG. 4B illustrates a consensus sequence of the F06 VI region, FIG. 4C illustrates all F06 454 reads, and FIG. 4D illustrates the relative abundance of the VI sequence.

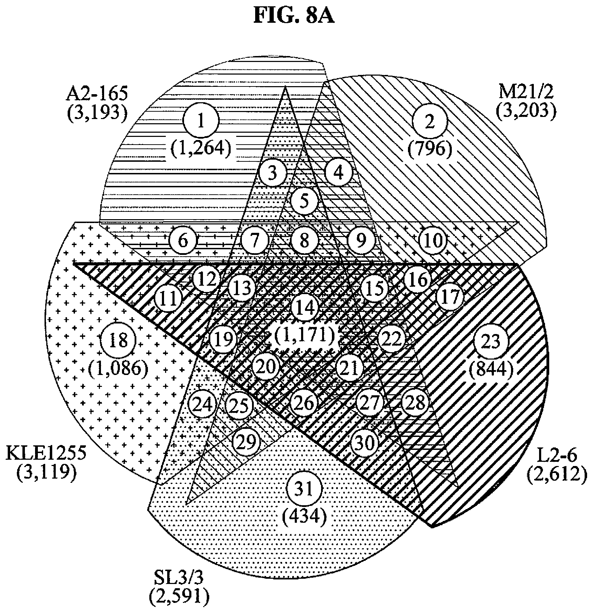

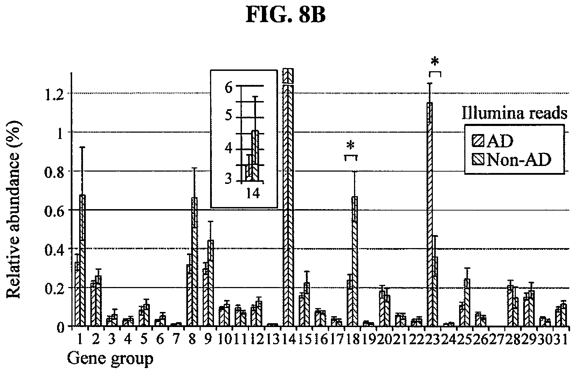

Five strains of *F. prausnitzii*, that is, A2-165, M21/2, L2-6, S3L/3, and KLE1255, have been sequenced at the genomic level through the Human Microbiome Project (HMP). Among the above strains, L2-6 has a very unique V1-V2 region, in particular, a VI region, in comparison to those of the four other strains, and closely matches the F06 clade (see FIGS. 4A through 4D). Although the V1-V2 region is a part of the 16S rRNA gene, an almost perfect match over the entire region indicates a very close relationship between L2-6 and F06 bacteria. By performing a cross-comparison of "15,825" predicted protein sequences in the five strains of *F. prausnitzii* using BLASTP and by removing overlapping genes, it was found that the *F. prausnitzii* pangenome was formed of "7,253" genes (see FIGS. 8A and 8B). The results indicated that the L2-6 strain had "855" unique genes and that the other strains had a similar number of unique genes. To compare the gut microbiomes and *F. prausnitzii* pangenome, eight samples (four samples each from the experiment group and the control group) were selected and subjected to whole-genome shotgun sequencing using the Illumina HiSeq 2000 platform, to obtain sequences of 552 Mb on average per sample. The eight samples were selected based on similar and high *F. prausnitzii* contents (19% to 36%). It was observed that when the Illumina reads were mapped to the *F. prausnitzii* pangenome, most (87.4%) of genes unique to L2-6 matched the reads from the atopic patients better than the reads from the control group (see FIGS. 8A and 8B). On the other hand, the genes unique to A2-165 and KLE1255 matched the reads from the non-atopic controls much better. Therefore, it was concluded that bacteria belonging to F06 are highly related to the L2-6 strain at the genomic level as well as at the V1-V2 region.

Change in metabolic functions of atopy microbiome

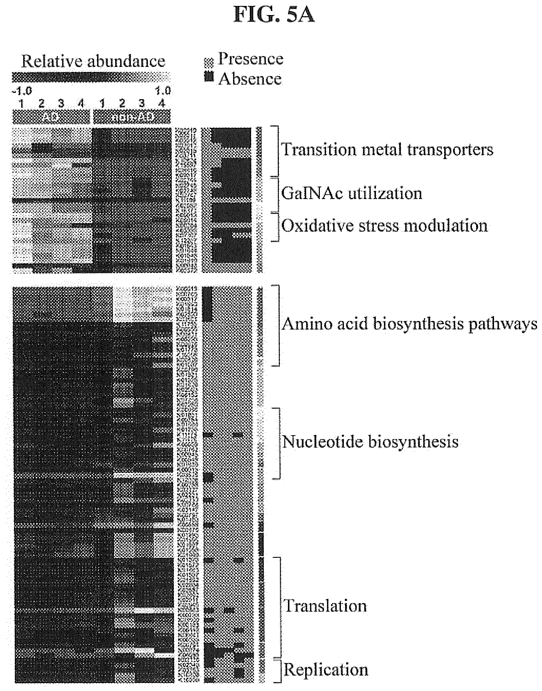

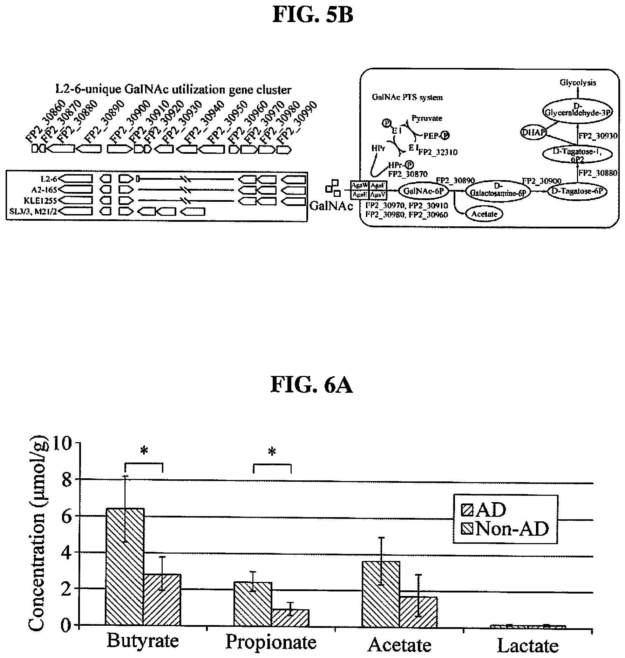

A metabolic change in the AD microbiomes was found using a method of mapping Illumina reads to the *F. prausnitzii* pangenome and KO database. The *F. prausnitzii* pangenome approach was effective due to the high *F. prausnitzii* content (19% to 36%) in the metagenome. Although the *F. prausnitzii* pangenome is formed of five strains, it is important that two biologically significant strains are included: A2-165, which is known to be lacking in Crohn disease, and L2-6, which was found to be associated with atopy in the present study. To interpret the mapping results, the *F. prausnitzii* pangenome was analyzed based on the KO database, and KOs ($P < 0.05$) with different numbers of matching reads between the experimental group and control group were collected. Among a total of "1,332" KOs assigned to the pangenome, "415" KOs showed a statistically significant difference between the experimental group and the control group, "122" KOs were increased in the experimental group, and "293" KOs were increased in the control group (see FIGS. 5A and 5B).

KOs that were increased in the AD microbiome include those with metabolic functions responsive to oxidative stress (for example, peroxiredoxin, DMSO reductase, and the *sufBC* operon), various transition metal transporters (for example, Fe, Ni, Zn, and Mn) that are involved in various pathways, and a function of transporting and performing catabolism of a major mucin component, that is, GalNAc (see FIGS. 5A and 5B). In contrast, genes with a decreased content in the AD microbiome included genes involved in the biosynthesis of various amino acids, nucleotides, peptidoglycans and cofactors (see FIGS. 5A and 5B). In the damaged gut environment, host-derived general nutrients, such as amino acids, cofactors, and coenzymes, as well as specific nutrients such as the mucin component GalNAc may be increased. An increase in general nutrients may result in a reduction in the biosynthetic gene content in the microbiome, and an increase in specific nutrients, such as GalNAc, may result in the selective increase in auxotrophic specialists or pathobionts that are capable of using the above materials. The above gut environment may further promote damage to the gut epidermis, which in turn, accelerates the dysbiosis of the gut microbiome. Among the five strains of *F. prausnitzii,* only L2-6 contains a gene cluster that utilizes GalNAc (see FIGS. 5A and 5B).

In the second method of mapping the Illumina reads to the KO database, "122" KOs among a total of "5,832" KOs showed differences between the atopy metagenome of the experimental group and metagenome of the control group. "66" KOs and "56" KOs were increased in the atopy metagenome and the metagenome of the control group, respectively. The results of the KO analysis were lower than those of the *F. prausnitzii* pangenomic analysis; however, this difference was because metagenomes other than *F. prausnitzii* were included. Similar to the *F. prausnitzii* pangenomic analysis, the KO analysis showed increases in a metal transporter, use of GalNAc, and oxidative stress response functions, and decreases in the biosynthesis of amino acids, nucleotides, and cofactors in the atopy metagenome. However, an increase in other genes such as genes related to use of L-fucose (L-fuculokinase and L-fucose isomerase) were found. L-fucose is released from mucin glycoproteins and used as a major nutrient component together with GalNAc by gut bacteria such as *Bacteroides thetaiotaomicron.* It was newly found that genes related to the glutathione S-transferase family, glucose-6-phosphate 1-dehydrogenase, glutamate decarboxylase, and glyoxylate and dicarboxylate metabolism were increased. Glutathione is a tripeptide of cysteine and glutamate and allows bacteria to maintain homeostasis while withstanding oxidative stress caused by reactive oxygen and nitrogen metabolites produced during inflammatory cascades. Glutathione-S-transferases perform a conjugation of a reduced glutathione to an endogenous compound, such as peroxidized lipids, for the purpose of detoxification. Glucose-6-phosphate 1-dehydrogenase levels have been shown to be increased *in E. coli under* oxidative stress, which leads to increases in antioxidant molecules of glutathione and nicotinamide adenine dinucleotide phosphate. Glutamate decarboxylase has been known to play an important role as an antioxidant in *E. coli* O157:H7 and *Saccharomyces cerevisiae.* Also, glyoxylate and dicarboxylate metabolism has been known to be involved in an antioxidation process. Thus, the above gene presence pattern further confirms that the gut epithelium tissues of a patient with AD are heavily damaged by inflammation (see FIGS. 5A and 5B).

A variety of auxotrophs and pathobionts were determined to be increased in the AD microbiome, and a diversity of GalNAc-specific IIA component genes (*agaF* and K02744) in a PTS system was examined. The results indicate that the above genes exist in a wide variety of forms. The increase in the genes that use mucin in different species indicates that a variety of auxotrophs and pathobionts other than the L2-6 strain flourish in the AD gut environment, which may be associated with the exacerbation and chronic nature of AD.

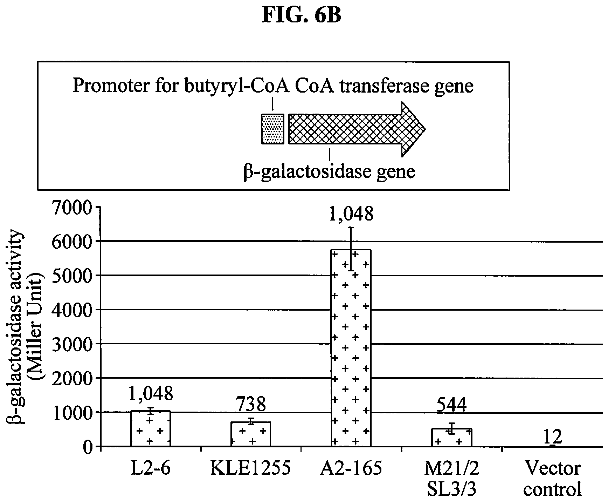

FIG. 6A is a graph illustrating analyses of SCFAs of the experimental group and control group, and FIG. 6B illustrates comparison of butyryl CoA : acetate CoA-transferase gene promoter activity from *F. prausnitzii* strains.

Increase in *F. prausnitzii* F06 subspecies and reduction in butyrate and propionate

The production of SCFAs in the gut microbiome has tremendous health benefits. To verify a difference in the production of SCFAs between the experimental AD group and control group, gas chromatographic-mass spectrometric analyses were conducted (see FIGS. 6A and 6B). Major SCFSs, for example, acetate, propionate, butyrate, and D- and L-lactate, were compared by selecting and pairing samples between the experimental group and control group that had similar total amounts of *F. prausnitzii* but that had significantly different F06 OTU levels (see FIGS. 6A and 6B). Because the total amount of *F. prausnitzii* in the samples was much higher than amounts of other SCFA producers, for example, *Eubacterium rectale, Eubacterium hallii,* and *Roseburia* species, the influence of other bacteria is likely insignificant and negligible, and any effect may be interpreted as the result of the influence of *F. prausnitzii.* The comparison results from the comparison of 12 pairs of samples revealed that samples with a low F06 level had higher amounts of butyrate and propionate than samples with a high F06 level (see FIGS. 6A and 6B). Such a great difference in the amounts of SCFAs is important data because the great difference suggests that the gut environment was very different between experimental group and control group. Differences other than the presence of AD, such as age, did not have a notable influence on the SCFA profile, and accordingly, it was concluded that the F06 level was the most important factor.

Because the L2-6 strain is related to the F06 clade and because the four other strains have the same butyrate biosynthesis pathway, the gene for the key enzyme butyryl CoA:acetate CoA-transferase was investigated to determine whether there was a difference in the expression of a pathway. The promoter of the above gene in each strain was synthesized and cloned into a promoter-probe vector upstream of the *lacZ* reporter gene, and gene expression was compared *in E. coli* (see FIGS. 6A and 6B). Although the above comparison was not performed in a *F. prausnitzii* host, the relative strengths of the promoters may be compared because the same conditions for the comparison were applied. The results show that the promoter of the A2-165 strain was significantly stronger than the promoters of the L2-6 strain and the other strains (see FIGS. 6A and 6B). In fact, the results are consistent with a study that reported a difference in butyrate production between the A2-165 and L2-6 strains. Moreover, the results indicate that butyrate levels in the samples were determined based on the composition of *F. prausnitzii* subspecies. Thus, dysbiosis of the *F. prausnitzii* associated with AD may result in the suppression of a high butyrate producer, such as A2-165 type bacteria, which may lead to a reduction in butyrate production.

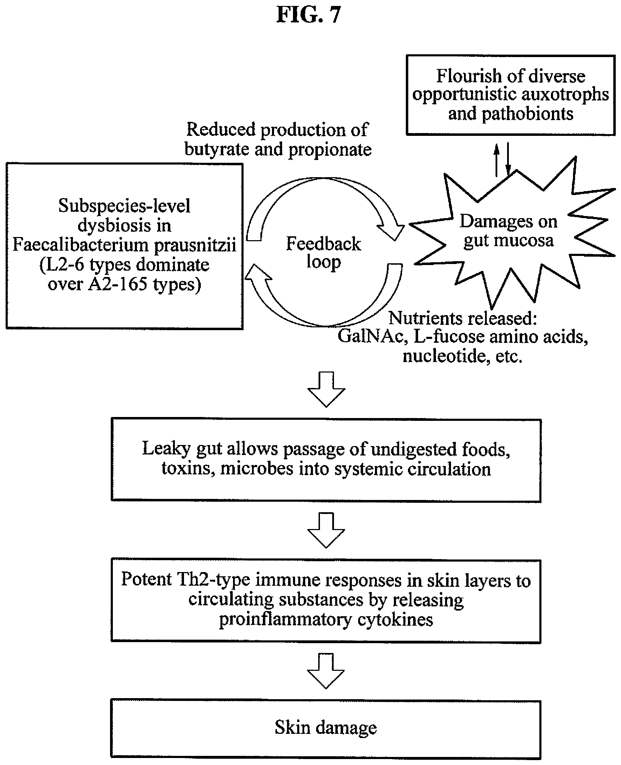

FIG. 7 illustrates a proposed model of AD associated with the dysfunction of *F. prausnitzii.*

*F. prausnitzii,* a dominant human intestinal bacterial species, has been shown to benefit gut health through the production of SCFAs. In particular, butyrate has an anti-inflammatory function and is used as a direct energy source by gut epidermal cells. Decreases in the level of *F. prausnitzii* and the production of SCFAs are associated with a Crohn disease. It has been reported that balance among subspecies of *F. prausnitzii,* in particular, changes between L2-6 and A2-165-type bacteria, affects the production of SCFAs. The AD metagenome was revealed to have an increased number of genes that utilize a mucin component, such as GalNAc and L-fucose, and various nutrients that may be released from damaged gut epithelial cells (see FIGS. 5A and 5B), which may indicate an increase in an inflammation in the gut epithelial cells and may correspond in a broad sense to a "leaky gut syndrome." When gut epithelial cells are damaged because of a dysbiosis in *F. prausnitzii* or other causes, further dysbiosis in *F. prausnitzii* and the growth of a variety of auxotrophs and pathobionts may be simultaneously promoted. As a result, a feedback loop may be formed between the dysbiosis in *F. prausnitzii* and the dysregulation of a gut epithelial inflammation.

## Claims

1.  A method of providing information, the method comprising:

    providing an isolated nucleic acid sample; and
    recognizing a nucleotide sequence comprising SEQ ID NO: 1 or SEQ ID NO: 2, wherein the recognizing of the nucleotide sequence is performed to diagnose atopic dermatitis (AD) .

2.  The method of claim 1, wherein the recognizing of the nucleotide sequence comprising SEQ ID NO: 1 or SEQ ID NO: 2 comprises detecting a subspecies of *Faecalibacterium prausnitzii.*

## Patentansprüche

1.  Verfahren zum Bereitstellen von Informationen, wobei das Verfahren umfasst:

    Bereitstellen einer isolierten Nukleinsäureprobe und
    Erkennen einer Nukleotidsequenz, die SEQ ID NO: 1 oder SEQ ID NO: 2 umfasst, wobei das Erkennen der Nukleotidsequenz durchgeführt wird, um atopische Dermatitis (AD) zu diagnostizieren.

2.  Verfahren nach Anspruch 1, wobei das Erkennen der SEQ ID NO: 1 oder SEQ ID NO: 2 umfassenden Nukleotidsequenz das Nachweisen einer Subspezies von *Faecalibacterium prausnitzii* umfasst.

**Revendications**

1.  Procédé de fourniture d'information, le procédé comprenant :

    la fourniture d'un échantillon d'acide nucléique isolé ; et
    la reconnaissance d'une séquence de nucléotide comprenant SEQ ID NO: 1 ou SEQ ID NO: 2, dans lequel la reconnaissance de la séquence de nucléotide est réalisée pour diagnostiquer la dermatite atopique (AD).

2.  Procédé selon la revendication 1, dans lequel la reconnaissance de la séquence de nucléotide comprenant SEQ ID NO: 1 ou SEQ ID NO: 2 comprend la détection d'une sous-espèce de *Faecalibacterium prausnitzii.*

# FIG. 1A

α diversity

## FIG. 1B

β diversity
(Bray-Curtis dissimilarity)

○Non-AD ○AD
PC1: 8.85%

**FIG. 1C**

Enterotypes

FIG. 1D

Ruminococcus

Parabacteroides

Faecalibacterium

Escherichia/Shigella

Relative abundance (%)

Age group

p = 0.0103

non AD -AD <1
non AD 1-6 -AD 1-6
non AD >6 -AD >6

## FIG. 2A

Histogram of test set accuracy

Frequency

79.92%
86.14%

70%    80%    90%    100%

Test set with top discriminatory OTUs
Test set with random OTUs

## FIG. 2B

| Rank | < 1 Taxon | OTUs | 1-6 Taxon | OTUs | 6 < Taxon | OTUs |
|---|---|---|---|---|---|---|
| 1 | Streptococcus | 375 | Lachnospiraceae | 488 | Faecalibacterium | 1084 |
| 2 | Bacteroides | 281 | Faecalibacterium | 418 | Lachnospiraceae | 422 |
| 3 | Veillonella | 276 | Lachnospiracea_incertae_sedis | 318 | Lachnospiracea_incertae_sedis | 329 |
| 4 | Faecalibacterium | 247 | Clostridium XVIII | 313 | Coprococcus | 252 |
| 5 | Lachnospiraceae | 208 | Clostridium XIVa | 179 | Bacteroides | 220 |
| 6 | Clostridium XI | 190 | Parabacteroides | 177 | Oscillibacter | 167 |
| 7 | Prevotella | 142 | Clostridium XI | 125 | Roseburia | 145 |
| 8 | Lachnospiracea_incertae_sedis | 127 | Bacteroides | 116 | Parabacteroides | 136 |
| 9 | Clostridium sensu stricto | 101 | Veillonella | 112 | Blautia | 124 |
| 10 | Enterobacteriaceae | 99 | Turicibacter | 82 | Clostridium XI | 121 |

# FIG. 3A

Phylogenetic tree of
Faecalibacterium prausnitzii OTUs

## FIG. 3B

OTU abundance

## FIG. 3C

F06 abundance

# FIG. 4A

V1-V2 regions in 16S rRNA gene of F. prausnitzii strains

# FIG. 4B

Consensus sequence - F06 V1

Consensus sequence - Most variable part in F06 V2

## FIG. 4C

All F06 454 reads (58,745)
blastn searched to L2-6 V1

Unmatched
2,469
(4.0%)

Matched

## FIG. 4D

EP 3 354 745 B1

# FIG. 5A

22

## FIG. 5B

## FIG. 6A

**FIG. 6B**

Promoter for butyryl-CoA CoA transferase gene

β-galactosidase gene

## FIG. 7

Flourish of diverse
opportunistic auxotrophs
and pathobionts

Reduced production of
butyrate and propionate

Subspecies-level
dysbiosis in
Faecalibacterium prausnitzii
(L2-6 types dominate
over A2-165 types)

Feedback
loop

Damages on
gut mucosa

Nutrients released:
GalNAc, L-fucose amino acids,
nucleotide, etc.

Leaky gut allows passage of undigested foods,
toxins, microbes into systemic circulation

Potent Th2-type immune responses in skin layers to
circulating substances by releasing
proinflammatory cytokines

Skin damage

EP 3 354 745 B1

**FIG. 8A**

A2-165
(3,193)

M21/2
(3,203)

1
(1,264)

2
(796)

3  4

5

6  7  8  9  10

12  13  15  16
11  14  17

18  19  22  23
(1,086)  (1,171)  (844)

20  21

24  25  26  27  28

29  30

KLE1255
(3,119)

31
(434)

L2-6
(2,612)

SL3/3
(2,591)

FIG. 8B